# EUROPEAN PATENT SPECIFICATION

(11) **EP 2 441 421 B1**
(45) Date of publication and mention of the grant of the patent: **19.07.2017**
(21) Application number: 10186970.9
(22) Date of filing: 08.10.2010
(51) Int. Cl.: A61F 13/56, A61F 13/62

(54) **Absorbent article with hook and loop fastening system**
Saugfähiger Artikel mit Klettverschlusssystem
Article absorbant doté d'un système de fixation à crochets et boucles

(43) Date of publication of application: 18.04.2012
(73) Proprietor: The Procter & Gamble Company, Cincinnati, OH 45202 (US)
(72) Inventor: Horn, Thomas Alexander, 65824 Schwalbach (DE); Homoelle, Dieter, 48607 Ochtrup (DE); Baldauf, Georg, 48366 Laer (DE)
(74) Representative: Heide, Ute

(56) References cited:
- WO-A1-2006/045118
- US-A1- 2006 080 810
- US-A1- 2010 175 825

## Description

### FIELD OF THE INVENTION

The invention relates to a absorbent article comprising a a hook and loop fastener wherein the female composite material element comprises a carrier and a knitted fabric adhesively attached onto the carrier. The knitted fabric has warp threads proceeding in the warp direction and loops incorporated therein suitable for connecting with hooks.

### BACKGROUND OF THE INVENTION

Hook and loop fasteners for absorbent articles and specifically for diapers are well know in the art. In one type of hook and loop fastener, the loop member comprises a carrier, such as a film layer, to which a knitted fabric is adhesively attached. For fastening the hook and loop member to each other, the hooks comprised by the hook member engage with the knitted fabric.

Typically, the loop member of the fastener is attached to the outer surface of the diaper in the front waist region (often referred to as "landing zone") while the hook members are attached to the back waist region. In use the hook fasteners are attached to the landing zone to attach the absorbent articles onto the wearer and to hold it in place around the waist and hip of the wearer. Hook and loop fasteners can be opened and closed multiple times without compromising the functionality of the fastener. In contrast to adhesive fasteners, hook and loop fasteners are unaffected by contact with skin creams or powders.

For use in absorbent articles, different requirements are asked of the loop members comprising a knitted fabric. The knitted fabric should, on the one hand, ensure sufficient interlocking with hooks and, on the other hand, be cost efficient to manufacture, thus have a basis weight as low as possible. Achieving a secure connection with hooks requires a sufficient number of freely moving loops and fibers of the knitted fabric, whose function must not be diminished by the adhesion of the knitted fabric onto the carrier. At the same time, the knitted fabric must be designed to be so stable and sufficiently connected adhesively to the carrier to avoid detaching and ripping even if the hook and loop fastener is used multiple times.

A hook and loop fastener with a loop member comprising a knitted fabric is known from WO 2006/045118 A1, which depicts an adhesive attachement of the knitted fabric onto a carrier in a lattice pattern having straight, perpendicularly intersecting, adhesive strips as a possible option.

Also from EP 1 997 942 A1 refers to a hook and loop fastener wherein the knitted fabric of the loop member is attached to a nonwoven carrier.

US 2006/080810 A1 relates to a knitted fabric female fastening portion for a mechanical fastener having a knitted fabric, an underlying substrate, and a bonding layer. The bonding layer has a first plurality of non-intersecting bond lines and a second plurality of non-intersecting bond lines which are combined to form a pattern of intersecting bond lines that define tessellating pattern elements.

Further hook and loop fastener are known from EP 1 579 779 A1 and EP 1 690 967 A1.

Despite the hook and loop fastener known in the art, there is still a need for improved hook and loop fastener where the adhesive attachment between the knitted fabric of the loop member onto the carrier is done in a cost efficient manner while ensuring sufficient engagement with the hook members to allow for proper and reliable attachment of the article onto the wearer.

### SUMMARY OF THE INVENTION

The invention refers to an absorbent article as defined in claim 1 and comprising a loop member for a hook and loop fastener, having a carrier and a knitted fabric laminated onto the carrier. The knitted fabric has warp threads in the warp direction (hereinafter also referred to as the longitudinal direction) and loops incorporated therein suitable for connecting with hooks. The carrier and the knitted fabric are adhesively attached to each other such that only a part of the surface area of the carrier facing towards the knitted fabric is covered with adhesive. The adhesive is applied in the form of a first plurality of adhesive stripes and a second plurality of adhesive stripes. The second plurality of adhesive stripes extends perpendicular to the first plurality of adhesive stripes and intersects the first plurality of adhesive stripes. The first plurality of adhesive stripes extends in warp direction. The distance between neighbouring adhesive stripes of the first plurality of stripes is greater than the distance between neighbouring warp threads.

### BRIEF DESCRIPTION OF THE DRAWINGS

These and other features, aspects and advantages of the present invention will become better understood with regard to the following description, appended claims, and accompanying drawings where:
- **Fig**. **1A**: a top view of a loop member according to the invention
- **Fig. 1B**: an enlarged view of a section of the loop member of Figure 1A
- **Figs**. **2A and 2B**: adhesive pattern according to the prior art(not showing the knitted fabric)
- **Fig. 2C**: a schematic drawing of an adhesive pattern of the present invention (not showing the knitted fabric)

### DETAILED DESCRIPTION OF THE INVENTION

"Absorbent article" refers to devices that absorb and contain body exudates, and, more specifically, refers to devices that are placed against or in proximity to the body of the wearer to absorb and contain the various exudates discharged from the body. Absorbent articles may include diapers, pants, training pants, adult incontinence undergarments, and the like. As used herein, the term "body fluids" or "body exudates" includes, but is not limited to, urine, blood, vaginal discharges, breast milk, sweat and fecal matter. Preferred absorbent articles of the present invention are diapers and refastenable pants.

"Comprise," "comprising," and "comprises" are open ended terms, each specifies the presence of what follows, e.g., a component, but does not preclude the presence of other features, e.g., elements, steps, components known in the art, or disclosed herein.

"Disposable" is used in its ordinary sense to mean an article that is disposed or discarded after a limited number of usage events over varying lengths of time, for example, less than about 20 events, less than about 10 events, less than about 5 events, or less than about 2 events. A disposable absorbent article is most often disposed after single use.

"Diaper" refers to an absorbent article generally worn by infants and incontinent persons about the lower torso so as to encircle the waist and legs of the wearer and that is specifically adapted to receive and contain urinary and fecal waste.

"Pant" or "training pant", as used herein, refer to disposable garments having a waist opening and leg openings designed for infant or adult wearers. A pant may be placed in position on the wearer by inserting the wearer's legs into the leg openings and sliding the pant into position about a wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened). While the terms "pant" or "pants" are used herein, pants are also commonly referred to as "closed diapers," "prefastened diapers," "pull-on diapers," "training pants," and "diaper-pants".

In practice, unintended opening of hook and loop fasteners has sometimes been observed if the hooks comprised by the hook member have been attached onto the loop member using only light contact/engaging strength and resulting in too few hooks engaging properly with the loop member. In such a case, there is the risk that number of hooks engaged with the loops of the knitted fabric is not sufficient to facilitate reliable fastening of the absorbent article onto the wearer. It must be taken into consideration that the application of baby diapers or comparable incontinence articles for adults is frequently carried out by helpers, wherein the person wearing the diaper often does not keep still, making application and fastening more difficult. If users can put on adult diapers by themselves, the problem moreover persists that they are often limited in their mobility and coordination.

Unintended opening of the fastening system may occur in longitudinal direction of the loop member as well as in transverse direction of the loop member. The longitudinal direction, as used herein, refers to the direction in which the warp threads extend. For many embodiments, the longitudinal direction of the loop member is coincident with the longitudinal direction of the absorbent article, onto which the loop member is attached. For the present invention, the longitudinal direction corresponds to the machine direction during manufacturing of the loop member.

In use, opening in the transverse direction is the direction in which the loop member is typically opened (and closed) when the absorbent article is applied onto a wearer. Especially for older babies and toddlers, it may happen that the wearer themselves open the absorbent article especially if the hook and loop fasteners can be readily opened with application of relatively small forces.

Also, in use, unwanted opening in the longitudinal direction can result from leg or waist movement of the wearer, which may induce peel forces in the longitudinal direction of the closed fastening system.

Thus, for reliable engagement of the hooks with the loops, the peel strengths of both the longitudinal direction and the transverse direction should be relatively high.

The present invention provides a loop member of a hook and loop fastener, which can be manufactured economically and which also shows good hook and loop fastening ability and reliability with regard to both, peel strength in the longitudinal direction and peel strength in the transverse direction.

The loop member of the present invention comprises a knitted fabric laminated onto a carrier. The knitted fabric has warp threads proceeding in the warp direction and loops incorporated therein suitable for connecting with hooks. The carrier and the knitted fabric are not connected with each other over the entire surface. At least in the central area of the loop member, the adhesive forms a pattern with perpendicularly intersecting, adhesive stripes. The pattern is applied on the carrier before the knitted fabric is attached to the carrier. A part of the adhesive stripes proceed parallel to the warp threads in the warp direction while the remaining part of the adhesive stripes extends perpendicular to the warp threads. The loop member forms the female (loop) member of a hook and loop fastener and is comprised by an absorbent article, such as a diaper. In one embodiment, the knitted fabric is adhesively attached directly to the outer cover of the absorbent article, such as a diaper, with the adhesive pattern of the present invention. Thus, in these embodiments the outer cover of the absorbent article serves a the carrier. Apparently, in these embodiments, the dimension of the carrier is considerably larger than the dimension of the knitted fabric (as the carrier will typically be the backsheet of the absorbent article). For embodiments wherein the knitted fabric is directly adhesively attached to the absorbent article, especially for any values and percentages given herein, the dimension and size of the loop member is defined by the size and dimensions of the knitted fabric.

The loop member of the hook and loop fastener is typically affixed to the front waist region of the absorbent article and the hook member is typically attached at the longitudinal side edges of the absorbent article in the back waist region. The hook and loop fastener ensures that the absorbent article is duly held in place around the waist of the wearer. Hook and loop fasteners can be opened and closed multiple times without compromising the functionality of the fastener. Achieving a secure connection with hooks requires a sufficient number of freely moving loops and fibers of the knitted fabric, whose function are not diminished by the adhesion of the knitted fabric onto the carrier. At the same time, the knitted fabric should stable and sufficiently connected adhesively to the carrier to avoid detaching and ripping even if the hook and loop fastener is used multiple times.

Though loop members with knitted fabrics adhesively attached to a carrier in a pattern of crossing adhesive lines are known in the art, in the adhesive pattern provided in the prior art generally the distance between neighbouring adhesive stripes running parallel to the warp threads is substantially equal to the distance beween neighbouring warp threads. However, in such adhesive attachment having an exact pattern repeat, all warp threads may be incorporated in adhesive attachment along the warp threads, resulting in relatively many loops and fibers not being available for engagement with the hooks.

According to the present invention, a knitted fabric is adhesively attached to the carrier by an adhesive pattern having a first plurality of adhesive stripes and a second plurality of adhesive stripes, the first and second plurality of adhesive stripes intersecting with each other. The first plurality of adhesive stripes is running generally parallel to the warp threads (i.e. is running in the longitudinal direction) of the knitted fabric, while the second plurality of adhesive stripes is running generally perpendicual to the warp threads (i.e. is running in the transverse direction), and thus, also generally perpendicular to the first plurality of adhesive stripes.

According to the present invention the knitted fabric is adhesively attached to the carrier such that the distance between two neighboring adhesive stripes of the first plurality of adhesive stripes is from four to eight times the distance between two neighbouring warp threads. Thereby, it is ensured that multiple warp threads are provided, which are only attached to carrier by the second plurality of adhesive stripes, which are running perpendicular to the warp thread direction. The distance between two neighbouring adhesive stripes is determined by the distance between the center of one adhesive stripe and the center of the neighbouring adhesive stripe. The measured distance from center to center two neighboring adhesive stripes of the first plurality of adhesive stripes proceeding in the warp direction is between 7 mm and 20 mm, preferably from 7 mm to 15 mm.

The width of the adhesive stripes of the first plurality of adhesive stripes may be from 0.5 mm to 5 mm, preferably from 0.5 to 1.5 mm and even more preferably from 0.5 mm to 1.2 mm. The width of the adhesive stripes of the second plurality of adhesive stripes may be from 0.5 mm to 5 mm, preferably from 0.5 to 1.5 mm and even more preferably from 0.5 mm to 1.2 mm. The width of the adhesive stripes of the second plurality of adhesive stripes may the the same as the width of the adhesive stripes of the first plurality of adhesive stripes or may be different from the width of the adhesive stripes of the first plurality of adhesive stripes. For the present invention, very small adhesive stripes having a width of less than 0.4 mm or less than 0.5 mm, are not considered to be comprised by the adhesive pattern of first and second plurality of adhesive stripes. Adhesive stripes of such width will not or only to a very limited degree contribute to an efficient attachment of the knitted fabric onto the carrier (a warp thread typically used for loop members on absorbent articles may have a width in the range of 1 mm to 2 mm). The same applies to very small adhesive dots (such as dots having a diameter of less 0.4 mm or less than 0.5 mm) on the carrier which may be due to manufacturing and processing shortcomings.

For the present inventon it is preferred that the first and second plurality of adhesive lines are continuous. However, in certain embodiments, the first and/or second plurality of adhesive stripes may be intermittent, i.e. they may be provided in the form of dotted lines. Thereby, fragments of adhesives stripes are provided which are interrupted by short adhesive free spaces. For such embodiments the average length of the fragements of adhesives stripes may be the same as the length of the spaces. Alternatively, the spaces between the fragments of the adhesive lines may be considerably shorter compared to the fragments of adhesive lines. E.g. the fragements of adhesive lines may -on average- be at least 2 times the length of the spaces.

Thereby, those warp threads, which are positioned at or in close proximity (i.e. close enough to have at least some minimal adhesive attachment) to an adhesive stripe of the first plurality of adhesive stripes, are relatively closely attached to the carrier. For these warp threads, the ability to engage with the hook members may be reduced compared to the warp threads, which are positioned more remote from an adhesive stripe of the first plurality of adhesive lines. The warp threads positioned more remote from an adhesive stripe of the first plurality of adhesive stripes are more readily available for easy engagement with the hook members. These warp threads are adhesively attached onto the carrier only by the second plurality of adhesive stripes. It should be noted that in these areas, not only the loops comprised by the knitted fabric can engage with the hooks but also other threads comprised by the knitted fabric. Generally, a relatively high percentage of the carrier surface covered by adhesive may reduce the ability of the knitted fabric to connect with hooks, because an unduly high number of loops and fibers may be, at least partially, adhesively connected to the carrier, making them unavailable for hook engagement. Also, the overall manufacturing cost increase with increased application of adhesive.

If the area of the carrier to be provided with adhesive is too small, the danger exists that the knitted fabric tears due to unequal strength distribution. Moreover, the integrity of the loop member (i.e. the adhesive connection between the knitted fabric and the carrier) can decrease to an extend that knitted fabric is separated from the carrier.

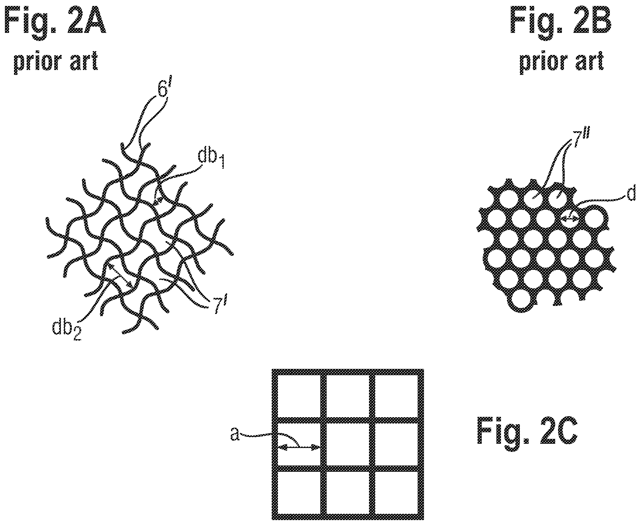

The best local hook and loop engagement can be expected in the center of the areas delimted and confined by the first and second plurality of adhesive stripes. In those areas the knitted fabric is relatively freely movable However, as those areas are always surrounded by the confining stripes of adhesive, where the knitted fabric is reliably attached to the carrier, tearing of of the knitted fabric off the carrier can be avoided. The first and second plurality of adhesive stripes may from a multi-rectangular pattern. In one embodiment, the first and second plurality of adhesive stripes forms a multi-quadratic pattern (see Fig. 2C).

Generally, the less area of the carrier is convered with adhesive, the more loops and fibers of the knitted fabric are available to freely engage with the hooks. Therefore, reducing the surface area of the carrier facing towards the knitted fabric and covered with adhesive typically allows for improved engagement with the hook member. However, reducing the surface area covered with adhesive also increases the risk of detachment and delamination of the knitted fabric from the carrier in use. The adhesive pattern of the present invention with the first and second plurality of adhesive stripes provdes improved peel strength compared to adhesive pattern of the prior art without the need to unduly reduce the surface area covered with adhesive. Thus, reliable and proper hook engagement is improved without increasing the risk of delamination of the knitted fabric from the carrier. For the present invention, the surface area of the carrier facing towards the knitted fabric and provided with adhesive typically is from 20% to 30% of the surface area of the carrier facing towards the knitted fabric and provided with the pattern of the first and second plurality of adhesive stripes (i.e. excluding the outer edges of the loop member provided with a frame, in embodiments where such a frame is used).

The distance between neighboring warp threads is significantly smaller compared to the distance of neighbouring adhesive stripes of the first plurality of adhesive stripes. Typically the distance between neighbouring warp threads is from 1 mm to 4 mm, preferably from 1 mm to 3 mm and more preferably from 1.2 mm to 2.2 mm. The knitted fabric may have connecting threads proceeding in a zigzag pattern in the warp direction that run over two neighboring or over several neighbouring warp threads. An increase of the basis weight can provide more loops to allow engagement with hooks. On the other side, basis weight knitted fabrics lead to reduced cost. Low basis weight knitted fabrics also result in the knitted fabric becoming more translucent, improving the visibility of a graphic provided on the carrier (or provided on the outer cover of the absorbent article, if the carrier itself is sufficiently translucent). Preferably, the basis weight of the knitted fabric is from 8 g/m² to 40 g/m², more preferably from 8 g/m² to 21 g/² and even more preferably from 10 g/m² to 18 g/m².

In one embodiment, the carrier comprises or consists of a film. The film may have a basis weight from 5 g/m² to 50 gm², preferably from 5 g/m² to 30 g/m² and more preferably from 10 g/m² to 25 g/m². Useful film are mono-layered films, as well as multi-layered films. The layers of the multilayered fim can be coextruded or laminated to each other. Suitable films are, for example, made of polyolefins, such as polyethylene, polypropylene, polyester, polyamide, and copolymers thereof. Carrier films are preferably relatively inexpensive. In some embodiments, the surface is suitable for printing (at least one one of the film surfaces). A nonwoven web, such as those disclosed in EP 1 997 942 A1, can alternatively be used as the carrier. In embodiments, wherein the knitted fabric is directly adhesively attached to the absorbent article, the backsheet film or the backsheet nonwoven of the absorbent article serves as the carrier.

The knitted fabric typically consists of polymer threads, wherein monofilament yarns and/or multi-filament yarns can be used to form the knitted fabric. The knitted fabric may, for example, consist of polypropylene, polyester, polyamide, or other synthetic materials that can be processed with textile industry-specific techniques.

As is known from EP 1 690 967 B1, the carrier and the knitted fabric can be adhesively attached to one another over the entire surface along the outer edge of the loop member (e.g. over a width of 1 mm to 10 mm, preferably from 4 mm to 10 mm, starting from the outer edges of the carrier and extending towards the center of the carrier), thus providing a frame around the pattern of the first and second plurality of intersecting adhesive stripes. Such a frame further redues the risk of detaching the knitted fabric from the carrier. Moreover, a frame may allow reducing the bonded area within the frame.

The loop member, may have a rectangular, elongated shape, wherein the hook member of a hook and loop fastener can be attached at different positions in order to adjust the waist circumference of the absorbent article to the waist circumference of the respective weare. However, other geometric shapes of the composite material element can be generally realized by customizing appropriately.

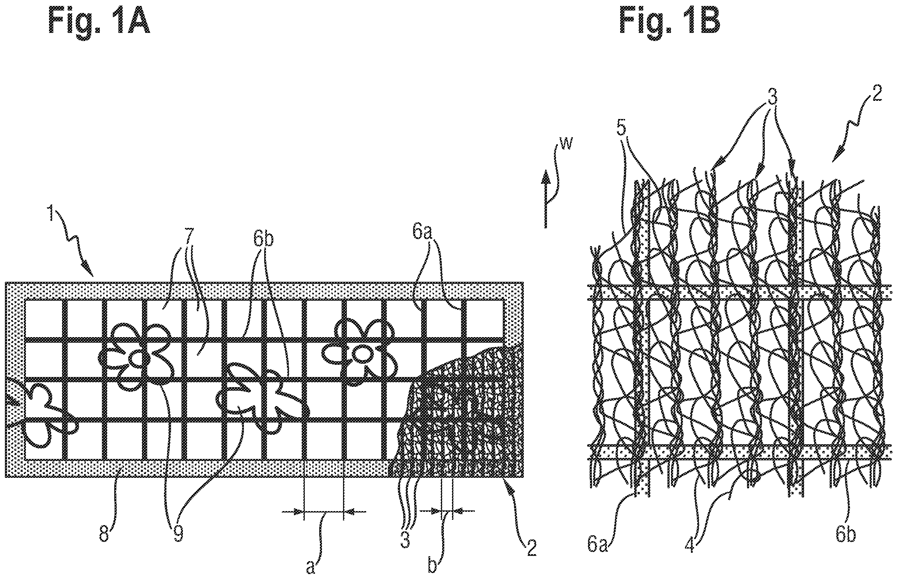

Fig. 1A and 1B show a loop member of a hook and loop fastener and which may be joined to the outer surface on the front waist region of an absorbent article, such as a diaper. The loop member consists of a carrier 1, such as a film, and a knitted fabric 2 adhesively attached onto the carrier 1. The knitted fabric 2 is formed from polymer threads as a warp-knitted fabric and comprises warp threads 3 proceeding in the warp direction W and loops 4 incorporated therein suitable for connecting with hooks. Parallel to one another, warp threads 3 are connected by connecting threads 5 running in a zigzag pattern.

The knitted fabric 2 is attached to the carrier 1 by an adhesive pattern, wherein the adhesive forms a pattern having straight, perpendicularly intersecting, adhesive stripes formed from a first plurality of adhesive stripes 6a provided substantially parallel to the direction W of the warp threads 3 and a second plurality of adhesive stripes 6b provided perpendicular to the first plurality of adhesive stripes 6a. Thus, a multi-square pattern is formed.

While individual warp threads 3 are held by the adhesive in the area of the first plurality of adhesive stripes 6a proceeding in the warp direction W, other warp threads 3 are provided between the adhesive stripes 6a proceeding in the warp direction W, are only held by the second plurality of adhesive stripes 6b proceeding in the transverse direction. Interlocking with hooks is possible in the area of these warp threads 3 within the areas 7, which are confined by the first and second plurality of adhesive stripes.At the same time the knitted fabric 2 is securely attached in the area of the second plurality of adhesive stripes 6a proceeding in the warp direction W, as well as in the areas, where the warp threads cross the second plurality of adhesive stripes. In order to prevent a detaching of the knitted fabric 2 from the carrier 1, a circumferential frame 8 may be provided at the outer edges of the loop member. The surface area of the carrier 1 provided with adhesive may be from 10% to 50%, preferably from 20% to 30% of the surface area of the carrier 1 facing towards the knitted fabric 2 and provided with the pattern of the first and second plurality of adhesive stripes 6a and 6b (i.e. the percentage excludes the area of the carrier 1 at the outer edges providing a frame 8, in embodiments where such a frame 8 is used).

The distance "a" between neighboring adhesive stripes of the first plurality of adhesive stripes 6a, is from 7 mm to 20 mm, preferably from 7 mm to 15 mm. The distance "b" between neighboring warp threads 3 is significantly smaller, and is from 1 mm to 4 mm, preferably from 1 mm to 3 mm, or from 1.2 mm to 2.2 mm. In the depicted exemplary embodiment, distance "a" determined from center to center between neighboring adhesive stripes of the first plurality of stripes 6a is 10 mm and distance b between neighboring warp threads 3 is 1.7 mm.

The distance between neighbouring adhesive stripes of the second plurality of adhesive stripes 6b may be the same as the distance between neighboring adhesive stripes of the first plurality of adhesive stripes 6a. However, in one embodiment of the invention, the distance between neighbouring adhesive stripes of the second plurality of adhesive stripes 6b is larger than the distance between neighboring adhesive stripes of the first plurality of adhesive stripes 6a. In one embodiment, the distance between neighbouring adhesive stripes of the second plurality of adhesive stripes 6b is from 10 %to 100% larger, or from 30% to 80%, than the distance between neighboring adhesive stripes of the first plurality of adhesive stripes 6a. In another embodiment, the distance between neighbouring adhesive stripes of the second plurality of adhesive stripes 6b is smaller than the distance between neighboring adhesive stripes of the first plurality of adhesive stripes 6a. In one embodiment, the distance between neighbouring adhesive stripes of the second plurality of adhesive stripes 6b is from 10 %to 100% smaller, or from 30% to 80%, than the distance between neighboring adhesive stripes of the first plurality of adhesive stripes 6a. Generally, in embodiments, wherein the distance between two neighbouring adhesive stripes of the first plurality of adhesive stripes is relatively high compared to the distance between two neighbouring warp threads (such as 6 to 10 times higher), the distance between two neighbouring adhesive stripes of the second plurality of adhesive stripes should not be smaller than the distance between two neighbouring adhesive stripes of the first plurality of adhesive stripes to avoid that the overall amount of surface area covered with adhesive does not become too low, resulting in negative effects such as detachment of the knitted fabric from the carrier.

As Fig. 1 shows, the carrier may be provided with a graphic 9 printed on the carrier. If a graphic is provided it should be visible through the translucent knitted fabric 2 from the outside of the absorbent article. In one embodiment, the basis weight of the knitted fabric is from 8 g/m² to 40 g/m², more preferably from 8 g/m² to 21 g/² even more preferably from 10 g/m² to 18 g/m².

In order to determine the efficiency of the hook and loop engagement comprising the loop member according to the invention, the maximum strength necessary for opening loop members having different adhesive patterns (i.e. the adhesive pattern applied between the carrier and the knitted fabric) as well as knitted fabrics having different basis weights was determined for two different, commercially available hooks.

For this, a square pattern of intersecting first and second pluralities of adhesive stripes according to the present invention and as illustrated in Fig. 2C was applied onto a carrier film (called "box" pattern in Tab. 1 to 4). As comparative examples, a pattern of intersecting wavy adhesive lines as shown in Fig. 2A (called "dog-bone" pattern in Tab. 1 to 4), and a pattern having dot-shaped areas as shown in Fig. 2B (called "dot" pattern in Tab. 1 to 4) has been applied on the identical carrier film.

In the box pattern according to the present invention, the distance "a" between neighboring adhesive stripes was 10 mm both for the first and second plurality of adhesive stripes (see Figure 2C). The width of the adhesive stripes was 1.2 mm. The surface area of the carrier facing towards the knitted fabric and covered with adhesive was 23% based on the total surface area of the carrier facing towards the knitted fabric (no surrounding frame has been applied in this embodiment).

The pattern depicted in Fig. 2A, having intersecting wavy-line adhesive stripes 6', is often referred to as a "dog bone" pattern due to the shape of the resulting areas 7'. For the dog bone pattern used in the comparative example, the surface area of the carrier covered with adhesive is 20% based on the total surface area of the carrier facing towards the knitted fabric (no surrounding frame has been applied in this embodiment). The smallest distance between two neighbouring wavy adhesive lines db₁ is 4.6 mm, distance db₂ as shown in Fig. 2A is 14.4 mm.

For the prior art pattern having dot-shaped areas 7" shown in Fig. 2B, the surface area of the carrier covered with adhesive was 25% based on the total surface area of the carrier facing towards the knitted fabric (no surrounding frame has been applied in this embodiment). The diameter d of the adhesive free cells 7" is 11.7 mm.

The carrier 1 was adhered to the knitted textile fabric using the adhesive pattern according to the invention as described above and the two comparison patterns as described above. The adhesive used was a one component polyurethane adhesive. Three different knitted fabrics were used for each adhesive pattern, wherein the basis weights of the different knitted fabric was 18 g/m², 21 g/m², and 25 g/m², respectively. For all examples, the knitted fabric was made of polyamide and the distance between two neighbouring warp threads is 1.7 mm.

The measurements been done using a) hooks from Aplix, type Aplix 962 as well as b) hooks CHK 01088 from 3M.

The carrier was a 3-layered polyethylene film having a basis weight of 17 g/m². The adhesive used was a one component polyurethane adhesive.

Peel strength measurements have been done both in the longitudinal direction of the knitted fabric as well as in the transverse direction of the knitted fabric according to ASTM method D 5170-98 (Reapproved 2004) titled "Standard Test Method for Peel Strength ("T" Method) of Hook and Loop Touch Fasteners", with the following changes versus the ASTM method D 5170-98 (Reapproved 2004): The width of the hook element is 25.4 mm and the length of the hook element is 13 mm. The hooks have been supplied in a roll with a width of 13 mm. These hooks have been adhesively attached onto a carrier to form a hook member, whereby the width of the hook roll became the length of the hook patch on the hook member. The hook element patches have been adhesively attached to a stripe of white copy paper of 90 g/m². The stripe had a dimension of 25.4 mm (width) x 210 mm (length). The hook element has been attached to the center of the paper stripe with the with the width of the hook patch of 25.4 mm coincident witthe the width of the stripe of paper.

A loop member (knitted fabric adhesively attached to the carrier) sample has been prepared having a width of 25.4 mm and a length of 100 mm. For the measurements of the peel strength in transverse direction, the warp direction of the knitted fabric corresponds to the width of the loop member. For the measurements of the peel strength in longitudinal direction, the sample has been prepared such that the warp direction of the knitted fabric corresponds to the length of the loop member.

The test procedure has been carried out following the procedure set out under item 8 of ASTM D5170-98 (Reapproved 2004) with the following change: Only the test configuration given as Sequence 1 in Fig. 2 of ASTM D 5170-98 (Reapproved 2004) has been measured.

The integrator average calculation method has been used (results see Table 3 and 4). In addition, the maximum peel strength, i.e. the value of the maximum peak (not mentioned in ASTM D 5170 98 (Reapproved 2004), is reported in Table 1 and 2.

For each embodiment, 10 samples have been prepared and measured and the mean value has been calculated for these 10 samples. The results are given in Tables 1 to 4.

**Table 1: Maximum peel strengths for hook Aplix 962**

| | 18 g knitted fabric | | | 21 g knitted fabric | | | 25 g knitted fabric | | |
|---|---|---|---|---|---|---|---|---|---|
| | Dot | Box | Dogbone | Dot | Box | Dogbone | Dot | Box | Dogbone |
| Max. peel strength in longitudinal direction [N/25.4 mm] | 2.0 | 3.4 | 2.4 | 3.7 | 4.4 | 3.8 | 4.1 | 4.4 | 3.1 |
| Max. peel strength in transverse direction [N/25.4 mm] | 2.1 | 4.0 | 2.6 | 5.3 | 5.6 | 4.7 | 4.3 | 4.0 | 5.0 |

**Table 2: Maximum peel strengths for hook hooks CHK 01088 from 3M**

| | 18 g knitted fabric | | | 21 g knitted fabric | | | 25 g knitted fabric | | |
|---|---|---|---|---|---|---|---|---|---|
| | Dot | Box | Dogbone | Dot | Box | Dogbone | Dot | Box | Dogbone |
| Max. peel strength in longitudinal direction [N/25.4 mm] | 2.4 | 5.3 | 2.8 | 4.7 | 4.0 | 4.3 | 5.2 | 6.1 | 5.2 |
| Max. peel strength in transverse direction [N/25.4 mm] | 2.8 | 7.0 | 3.9 | 6.0 | 5.7 | 5.9 | 5.4 | 6.1 | 5.2 |

**Table 3: Integrator average peel strengths for hook Aplix 962**

| | 18 g knitted fabric | | | 21 g knitted fabric | | | 25 g knitted fabric | | |
|---|---|---|---|---|---|---|---|---|---|
| | Dot | Box | Dogbone | Dot | Box | Dogbone | Dot | Box | Dogbone |
| Integrator average peel strength in longitudinal direction [N/25.4 mm] | 0.7 | 0.9 | 0.5 | 0.9 | 1.2 | 0.8 | 1.0 | 1.1 | 1.1 |
| Integrator average peel strength in transverse direction [N/25.4 mm] | 1.1 | 1.5 | 0.8 | 1.8 | 1.9 | 1.5 | 1.2 | 1.3 | 1.6 |

**Table 4: Integrator average peel strengths for hook hooks CHK 01088 from 3M**

| | 18 g knitted fabric | | | 21 g knitted fabric | | | 25 g knitted fabric | | |
|---|---|---|---|---|---|---|---|---|---|
| | Dot | Box | Dogbone | Dot | Box | Dogbone | Dot | Box | Dogbone |
| Integrator average peel strength in longitudinal direction [N/25.4 mm] | 0.6 | 1.5 | 0.9 | 1.3 | 0.9 | 1.3 | 1.1 | 1.2 | 1.8 |
| Integrator average peel strength in transverse direction [N/25.4 mm] | 1.1 | 2.2 | 1.5 | 1.8 | 1.9 | 1.7 | 1.5 | 1.9 | 1.8 |

As can be seen from Tables 1 to 4, higher strengths are needed to open the hook and loop fastener when the inventive adhesive pattern is used (both in the longitudinal and transverse direction). Thus, the fastening system enables more reliable fastening of absorbent articles onto a wearer compared to fastening systems of the prior art, especially in embodiments wherein the knitted fabric has a relatively low basis weight..

As can be seen from the data provided in Tables 1 to 4, the adhesive pattern of the present invention with the first and second plurality of adhesive stripes provdes improved peel strength compared to adhesive pattern of the prior art without the need to unduly reduce the surface area covered with adhesive. Thus, reliable and proper hook engagement is improved without increasing the risk of delamination of the knitted fabric from the carrier.

### Absorbent articles comprising the loop member

In the following, a diaper will be described as one example of an absorbent article of the present invention. It is however understood, that the other disposable absorbent articles are also encompassed by the present invention, such as refastenable pants, and training pants. Preferred absorbent articles of the present invention are disposable absorbent articles, such as disposable diapers.

The diaper generally may comprise a chassis and an absorbent core disposed in the chassis.

The chassis of the diaper comprises the main body of the diaper. The chassis may comprise an outer covering including a topsheet, which may be liquid pervious, and/or a backsheet, which may be liquid impervious. The absorbent core may be encased between the topsheet and the backsheet. Diaper also comprises an acquisition system disposed between the topsheet and the wearer facing side of the absorbent core. The acquisition system may be in direct contact with the absorbent core. The chassis may also include side panels, elasticized leg cuffs, and an elastic waist feature.

The leg cuffs and the elastic waist feature may each typically comprise elastic members. One end portion of the diaper is configured as a front waist region of the diaper. An opposite end portion of the diaper is configured as a back waist region. An intermediate portion of the diaper is configured as a crotch region, which extends longitudinally between the first and second waist regions and. The waist regions and may include elastic elements such that they gather about the waist of the wearer to provide improved fit and containment. The crotch region is that portion of the diaper which, when the diaper is worn, is generally positioned between the wearer's legs.

The diaper has a longitudinal axis (extending through the front and back waist region and through the crotch region) and its transverse axis (extending only through the crotch region). The periphery of the diaper is defined by the outer edges of the diaper in which the longitudinal edges run generally parallel to the longitudinal axis of the diaper and the end edges run between the longitudinal edges generally parallel to the transverse axis.

In order to keep the diaper in place about the wearer, at least a portion of the front waist region may be attached to at least a portion of the back waist region by a fastening system to form leg opening(s) and an article waist. According to the present invention, the diaper comprises a hook and loop fastening system with the loop member described above. The fastening system comprises hook members attached to the back waist region and at least one loop member attached to the front waist region. In one embodiment, the hook member is attached adjacent the longitudinal edges in the back waist region on both sides of the diaper. The hook members may be attached such a backing of the hook member is attached adjacent the longitudinal edges in the back waist region and the hooks (typically provided on a hook patch) are extending laterally outward beyond the longitudinal edges.

In one embodiment of the present invention, the loop member is attached to the outer surface of the absorbent article such that the warp threads extend along the longitudinal axis of the absorbent article or at a slight angle to the longitudinal axis (preferably not more than 20° or not more than 10°).

In one embodiment, the knitted fabric is adhesively attached to the outer cover of the absorbent article, such as a diaper, with the adhesive pattern of the present invention. Thus, in these embodiments the outer cover of the absorbent article serves a the carrier. Apparently, in these embodiments, the dimension of the carrier is considerably larger than the dimension of the knitted fabric (as the carrier will typically be the backsheet of the absorbent article). For embodiments wherein the knitted fabric is directly adhesively attached to the absorbent article, especially for any values and percentages given herein, the dimension and size of the loop member is defined by the size and dimensions of the knitted fabric. Also in those embodiments, the warp direction of the knitted fabric (i.e. the longitudinal direction of the knitted fabric) is coincident with longitudinal direction of the absorbent article.

The loop member of the present invention may also be used to attach a absorbent insert onto an outer cover of an absorbent article, such as a diaper. In such embodiments, the absorbent insert typically comprises an absorbent core, a topsheet and a backsheet. The absorbent insert may also comprise an acquisition system placed between the absorbent core and the topsheet. Also, in such embodiments, the chassis does not comprise a topsheet (as the topsheet is provided by the absorbent insert) but comprises an additional outer cover backsheet. The absorbent insert is attached to the chassis by providing one component of the fastening system (i.e. the hook member or the loop member) on the backsheet of the absorbent insert on the surface facing towards the chassis outer cover backsheet in use and providing the other component of the fastening system (i.e. the loop member or the hook member complemantary to the fastening component of the absorbent insert) on the surface of the chassis outer cover backsheet facing towards the absorbent insert in use. In such embodiments, the outer cover may be reusable while the absorbent insert is disposable.

In still another embodiment, the loop member of the present invention may be used as component for a fastening system provided for disposal on an absorbent article, such as a diaper.

## Claims

1. An absorbent article comprising a loop member for a hook and loop fastener, having a carrier (1) and a knitted fabric (2) laminated onto the carrier (1), wherein the knitted fabric (2) has warp threads (3) in the warp direction (W) and loops (4) incorporated therein suitable for connecting with hooks, wherein the carrier (1) and the knitted fabric (2) are adhesively attached to each other such that only a part of the surface area of the carrier facing towards the knitted fabric is covered with adhesive, the adhesive being in applied in the form of a first plurality of adhesive stripes (6a) and a second plurality of adhesive stripes (6b), the second plurality of adhesive stripes (6b) extending perpendicular to the first plurality of adhesive stripes (6a) and intersecting the first plurality of adhesive stripes (6a), the first plurality of adhesive stripes extending in warp direction (W), **characterized in that** the distance between neighbouring adhesive stripes of the first plurality of stripes (6a) is greater than the distance between neighbouring warp threads (3) with the distance between two neighbouring adhesive stripes of the first plurality of adhesive stripes (6a) being from four to eight times the distance between two neighbouring warp threads and **in that** the distance (b) between neighboring warp threads (3) is between 1 mm and 4 mm;
wherein first and second plurality of adhesive stripes (6a, 6b) form a multi-square pattern (7), and the distance (a) between neighboring adhesive strips (6a) proceeding in the warp direction (W) is between 7 mm and 20 mm,
and the surface area of the carrier (1) facing towards the knitted fabric (2) being between 20% to 30% based on the surface area of the carrier (1) covered with the pattern of first and second plurality of adhesive stripes (6a, 6b).

2. The absorbent article according to any of the preceding claims, wherein the distance (b) between neighboring warp threads (3) is between 1 mm and 3 mm, preferably between 1.2 mm and 2.2 mm.

3. The absorbent article according to any of the preceding claims, wherein the basis weight of the knitted fabric (2) is from 8 g/m² to 21 g/m².

4. The absorbent article according to any of the preceding claims, wherein the carrier (1) is a film.

5. The absorbent article according to any of the preceding claims, wherein the carrier (1) and the knitted fabric (2) are adhesively adhered to each other over the entire surface along the outer edges of the loop member to form and adhesive frame (8).

6. The absorbent article according to any of the preceding claims, wherein the loop member is attached to the outer surface of the absorbent article in the front waist area.

7. The absorbent article according to claim 6, wherein the loop member is rectangular and wherein the transverse dimension of the loop member is at least 3 times the longitudinal dimension of the loop member.

8. The absorbent article according to any of the preceding claims, the loop member is useful for fastening an absorbent insert onto an outer cover of an absorbent article.

9. The absorbent article according to any of the preceding claims, wherein the loop member is useful for fastening the absorbent article in a disposal configuration, and wherein the disposal configuration is a folded or rolled configuration.

10. The absorbent article according to any of the preceding claims, wherein the knitted fabric (2) is directly attached to the backsheet of the absorbent article such that the backsheet serves as the carrier (1).

## Patentansprüche

1. Absorptionsartikel, ein Schlaufenelement für einen Klettverschluss umfassend, mit einem Träger (1) und einem auf den Träger (1) laminierten Maschenstoff (2), wobei der Maschenstoff (2) Kettfäden (3) in der Kettrichtung (W) und darin eingearbeitete Schlaufen (4) aufweist, die für eine Verbindung mit Haken geeignet sind, wobei der Träger (1) und der Maschenstoff (2) auf solche Weise haftend aneinander gebunden sind, dass nur ein Teil der Oberfläche des Trägers, die dem Maschenstoff zugewandt ist, mit Klebstoff beschichtet ist, wobei der Klebstoff in Form einer ersten Vielzahl von Klebestreifen (6a) und einer zweiten Vielzahl von Klebestreifen (6b) aufgebracht ist, wobei die zweite Vielzahl von Klebestreifen (6b) senkrecht zur ersten Vielzahl von Klebestreifen (6a) verläuft und die erste Vielzahl von Klebestreifen (6a) kreuzt, wobei die erste Vielzahl von Klebestreifen in Kettrichtung (W) verläuft, **dadurch gekennzeichnet, dass** der Abstand zwischen einander benachbarten Klebestreifen der ersten Vielzahl von Streifen (6a) größer ist als der Abstand zwischen einander benachbarten Kettfäden (3), wobei der Abstand zwischen zwei einander benachbarten Klebestreifen der ersten Vielzahl von Klebestreifen (6a) vier bis acht Mal so groß ist wie der Abstand zwischen zwei einander benachbarten Kettfäden, und dass der Abstand (b) zwischen einander benachbarten Kettfäden (3) zwischen 1 mm und 4 mm beträgt;
wobei die erste und die zweite Vielzahl von Klebestreifen (6a, 6b) eine Muster aus vielen Quadraten (7) bildet,
und der Abstand (a) zwischen einander benachbarten Klebestreifen (6a), die in der Kettrichtung (W) verlaufen, zwischen 7 mm und 20 mm beträgt,
und die Oberfläche des Trägers (1), die dem Maschenstoff (2) zugewandt ist, bezogen auf die Oberfläche des Trägers (1), die mit dem Muster aus der ersten und der zweiten Vielzahl von Klebestreifen (6a, 6b) bedeckt ist, zwischen 20 % und 30 % ausmacht.

2. Absorptionsartikel nach einem der vorstehenden Ansprüche, wobei der Abstand (b) zwischen einander benachbarten Kettfäden (3) zwischen 1 mm und 3 mm, vorzugsweise zwischen 1,2 mm und 2,2 mm beträgt.

3. Absorptionsartikel nach einem der vorstehenden Ansprüche, wobei das Basisgewicht des Maschenstoffs (2) 8 g/m² bis 21 g/m² beträgt.

4. Absorptionsartikel nach einem der vorstehenden Ansprüche, wobei der Träger (1) eine Folie ist.

5. Absorptionsartikel nach einem der vorstehenden Ansprüche, wobei der Träger (1) und der Maschenstoff (2) über der gesamten Oberfläche entlang der äußeren Ränder des Schlaufenelements aneinander geklebt sind, um einen Kleberahmen (8) zu bilden.

6. Absorptionsartikel nach einem der vorstehenden Ansprüche, wobei das Schlaufenelement im vorderen Taillenbereich an der Außenfläche des Absorptionsartikels befestigt ist.

7. Absorptionsartikel nach Anspruch 6, wobei das Schlaufenelement rechteckig ist, und wobei die Querabmessung des Schlaufenelements mindestens 3 Mal so groß ist wie die Längsabmessung des Schlaufenelements.

8. Absorptionsartikel nach einem der vorstehenden Ansprüche, wobei das Schlaufenelement für die Befestigung eines absorbierenden Einsatzes an einem Außenmantel eines Absorptionsartikels nützlich ist.

9. Absorptionsartikel nach einem der vorstehenden Ansprüche, wobei das Schlaufenelement für die Befestigung des Absorptionsartikels in einer Entsorgungskonfiguration nützlich ist, und wobei die Entsorgungskonfiguration eine gefaltete oder aufgerollte Konfiguration ist.

10. Absorptionsartikel nach einem der vorstehenden Ansprüche, wobei der Maschenstoff (2) direkt an die Unterschicht des Absorptionsartikels gebunden ist, so dass die Unterschicht als Träger (1) dient.

## Revendications

1. Article absorbant comprenant un élément de boucle pour un fermoir à crochets et boucles, comportant un support (1) et un tissu à mailles (2) stratifié sur le support (1), dans lequel le tissu à mailles (2) a des fils de chaîne (3) dans le sens chaîne (W) et des boucles (4) incorporées en son sein, appropriées pour un raccordement avec des crochets, dans lequel le support (1) et le tissu à mailles (2) sont fixés de manière adhésive l'un à l'autre de telle sorte que seule une partie de la superficie du support faisant face au tissu à mailles est couverte d'adhésif, l'adhésif étant appliqué sous la forme d'une première pluralité de bandes adhésives (6a) et d'une deuxième pluralité de bandes adhésives (6b), la deuxième pluralité de bandes adhésives (6b) s'étendant perpendiculaire à la première pluralité de bandes adhésives (6a) et croisant la première pluralité de bandes adhésives (6a), la première pluralité de bandes adhésives s'étendant dans le sens chaîne (W), **caractérisé en ce que** la distance entre des bandes adhésives voisines de la première pluralité de bandes (6a) est plus grande que la distance entre des fils de chaîne voisins (3), la distance entre deux bandes adhésives voisines de la première pluralité de bandes adhésives (6a) étant de quatre à huit fois la distance entre deux fils de chaîne voisins, et **en ce que** la distance (b) entre des fils de chaîne voisins (3) est comprise entre 1 mm et 4 mm ;
dans lequel les première et deuxième pluralités de bandes adhésives (6a, 6b) forment un motif à plusieurs carrés (7),
et la distance (a) entre des bandes adhésives voisines (6a) s'étendant dans le sens chaîne (W) est comprise entre 7 mm et 20 mm,
et la superficie du support (1) faisant face au tissu à mailles (2) est comprise entre 20 % et 30 % sur la base de la superficie du support (1) couverte par le motif des première et deuxième pluralités de bandes adhésives (6a, 6b).

2. Article absorbant selon l'une quelconque des revendications précédentes, dans lequel la distance (b) entre des fils de chaîne voisins (3) est comprise entre 1 mm et 3 mm, de préférence entre 1,2 mm et 2,2 mm.

3. Article absorbant selon l'une quelconque des revendications précédentes, dans lequel la masse surfacique du tissu à mailles (2) va de 8 g/m² à 21 g/m².

4. Article absorbant selon l'une quelconque des revendications précédentes, dans lequel le support (1) est un film.

5. Article absorbant selon l'une quelconque des revendications précédentes, dans lequel le support (1) et le tissu à mailles (2) sont fixés par adhérence l'un à l'autre sur la totalité de la surface le long des bords externes de l'élément de boucle pour former un cadre adhésif (8).

6. Article absorbant selon l'une quelconque des revendications précédentes, dans lequel l'élément de boucle est fixé à la surface externe de l'article absorbant dans la zone de ceinture avant.

7. Article absorbant selon la revendication 6, dans lequel l'élément de boucle est rectangulaire et dans lequel la dimension transversale de l'élément de boucle vaut au moins 3 fois la dimension longitudinale de l'élément de boucle.

8. Article absorbant selon l'une quelconque des revendications précédentes, dans lequel l'élément de boucle est utile pour fixer un insert absorbant sur une couverture externe d'un article absorbant.

9. Article absorbant selon l'une quelconque des revendications précédentes, dans lequel l'élément de boucle est utile pour fixer l'article absorbant dans une configuration de mise au rebut, et dans lequel la configuration de mise au rebut est une configuration pliée ou roulée.

10. Article absorbant selon l'une quelconque des revendications précédentes, dans lequel le tissu à mailles (2) est directement fixé à la feuille de fond de l'article absorbant de telle sorte que la feuille de fond sert de support (1).
